# EUROPÄISCHE PATENTSCHRIFT

(11) **EP 0 439 710 B1**
(45) Veröffentlichungstag und Bekanntmachung des Hinweises auf die Patenterteilung: **08.06.1994**
(21) Anmeldenummer: 90122400.6
(22) Anmeldetag: 23.11.1990
(51) Int. Cl.: A23F 5/20

(54) **Verfahren zur Entcoffeinierung von Rohkaffee**
Process for decaffeinating green coffee
Procédé de décaféination de café vert

(30) Priorität: 10.01.1990 DE 4000474
(43) Veröffentlichungstag der Anmeldung: 07.08.1991
(73) Patentinhaber: KOHLENSÄURE-WERKE RUD. BUSE GMBH & CO., D-53557 Bad Hönningen (DE)
(72) Erfinder: Ben-Nasr, Hedi, Dr., W-4650 Gelsenkirchen-Resse (DE); Coenen, Hubert, Dr., W-4300 Essen 1 (DE)
(74) Vertreter: Liesegang, Roland, Dr.-Ing.

(56) Entgegenhaltungen:
- EP-A- 0 247 999
- EP-A- 0 250 845
- EP-A- 0 313 921
- CH-A- 239 206
- DE-A- 2 450 978
- DE-A- 2 638 383
- DE-A- 3 713 953
- FR-E- 12 612

## Beschreibung

Die Erfindung betrifft ein Verfahren zur Entcoffeinierung von Rohkaffee nach dem Oberbegriff von Patentanspruch 1, mit dem man einen den Definitionen entsprechenden coffeinarmen oder coffeinfreien Rohkaffee erhält. Andererseits erhält man nach dem erfindungsgemäßen Verfahren als Nebenprodukt kristallinreines Coffein, welches in der Pharmazie und Getränkeindustrie Verwendung finden kann. Ein derartiges Verfahren ist aus der EP-A 250 845 (= DE-A 3 713 953) bekannt.

Bohnenkaffee wird häufig wegen seines Gehaltes an Coffein von vielen Menschen nicht gut vertragen. Viele Verfahren sind daher mit dem Ziel entwickelt worden, dem Rohkaffee das Coffein zu entziehen und gleichzeitig die Entfernung der anderen Rohkaffee-Inhaltstoffe, die bei der Röstung für die Aromastoffbildung erforderlich sind zu vermeiden. Das Coffein wird dem Rohkaffee entzogen, weil bei der Entcoffeinierung von Röstkaffee ein Aromaverlust nicht zu vermeiden ist.

Bei einem in der Praxis angewendeten Entcoffeinierungsverfahren wird nach der Vorbehandlung des Rohkaffees, z.B. durch Aufschließen der Kaffeebohnen mit Wasserdampf bei erhöhter Temperatur, einer Flüssigextraktion der Kaffeebohnen mit Lösungsmitteln, z.B. mit Methylenchlorid oder Essigester, das Lösungsmittel aus dem Rohkaffee durch Abdampfen entfernt und danach der feuchte Rohkaffee getrocknet. Bei diesem bekannten Verfahren ist nicht auszuschließen, daß Lösungsmittelreste im Bohnenkaffee verbleiben, und daß eine gewisse Denaturierung des Rohkaffees eintritt.

Es wurden auch Verfahren vorgeschlagen, die andere Lösungsmittel verwenden, die nicht aus dem entcoffeinierten Rohkaffee entfernt werden müssen. Praktische Bedeutung haben vor allem die Verfahren erlangt, die Wasser, überkritisches Kohlendioxid, flüssiges Kohlendioxid oder aus Kaffee stammende höhere organische Fettsäuren ( Kaffeeöl ) als Lösungsmittel verwenden. Bei diesen Verfahren sind lange Extraktionszeiten erforderlich, damit eine ausreichende Entcoffeinierung erzielt wird. Dies ist darauf zurückzuführen, daß das Coffein erst an die Oberfläche der Kaffeebohnen diffundieren muß, damit es vom Lösungsmittel aufgenommen wird. Dieser Vorgang verlangsamt sich zunehmend mit abnehmender Coffeinkonzentration in den Kaffeebohnen. Bei vielen dieser bekannten Verfahren wird das Lösungsmittel während der Extraktion im Kreislauf gefahren, wodurch hohe Investitions- und Energiekosten verursacht werden.

Aus der DE-OS 3713953 A1 ist ein Verfahren zur Entcoffeinierung von Rohkaffee bekannt, daß die genannten Nachteile weitestgehend vermeidet. Dabei wird der auf einen hohen Wassergehalt von 35 bis 50 Gew.-% angefeuchtete Rohkaffee einige Minuten bis einige Stunden bei einer Temperatur von 20 bis 80 °C unter einer Gasatmosphäre von 75 bis 300 bar gehalten und dabei gegebenenfalls umgerührt. Anschließend wird schlagartig oder in wenigen Minuten unter Vermeidung des Einfrierens auf 1 bar bis p_{c} ( p_{c} = kritischer Druck des verwendeten Gases ) entspannt. Der Rohkaffee wird mit Wasser oder mit dem überkritischen Gas zur selektiven Extraktion des Coffeins gespült und das Verfahren ggf. mehrfach wiederholt. Anschließend wird der Rohkaffee in einer Zentrifuge vorgetrocknet und geröstet. Das Coffein wird in bekannter Weise aus dem Spülmittel gewonnen.
Bei diesem Verfahren ist ein aufwendiger und kostenintensiver mehrmaliger Aufbau der Druckatmosphäre erforderlich.

Aus der DE-A-24 50 978 ist ferner die Verwendung von Inertgasen als Druckmittel bei der Entcoffeinierung von Kaffee bekannt, und in der DE-A- 26 38 383 sind bevorzugte Temperatur- und Druckbereiche von 60° bis 95°C bzw. von 120 bis 300 bar zur Extraktion von Coffein aus einer wässrigen Extraktionslösung mit überkritischem Kohlendioxid angegeben.

Der Erfindung liegt die Aufgabe zugrunde, ein einfaches, betriebssicheres und wirtschaftlisches Verfahren zur Entcoffeinierung von Rohkaffee zu schaffen, das einen hohen Entcoffeinierungsgrad gewährleistet, eine Denaturierung der weiteren Bestandteile des Rohkaffees vermeidet und eine Gewinnung des Coffeins gestattet.

Die der Erfindung zugrundeliegende Aufgabe wird durch ein Verfahren zum Entcoffeinieren von Rohkaffee mit den Merkmalen von Patentanspruch 1 gelöst.

Der überraschende Erfolg dieses Druckwechselverfahrens ist u.a. darauf zurückzuführen, daß eine Reihe von Wirkungen auftreten, die sich in vorteilhafter Weise ergänzen. So wird durch das aufgenommene Wasser in den Zellen der Rohkaffeebohnen eine Wasser-Coffein-Lösung gebildet, die die Extraktion des Coffeins erleichtert. Die Extraktion anderer Stoffe, insbesondere derjenigen, die für die Aromabildung beim Rösten erforderlich sind, wird auf ein Minimum reduziert.
Durch das schnelle Entspannen erfolgt eine starke Volumenvergrößerung des in die Rohkaffeebohnen vorher eindiffundierten Gases, wodurch ein Austreibeffekt der Coffein-Wasser-Lösung an die Oberfläche der Rohkaffeebohnen bewirkt wird. Das Coffein wird selektiv von der den Rohkaffee umgebenden Flüssigphase aufgenommen und abtransportiert. Das Coffein wird deswegen selektiv aufgenommen, weil die Flüssigphase mit den anderen Rohkaffee-Inhaltstoffen infolge der vorteilhaften Prozessführung unter den Verfahrensbedingungen gesättigt, aber weitestgehend coffeinfrei ist.

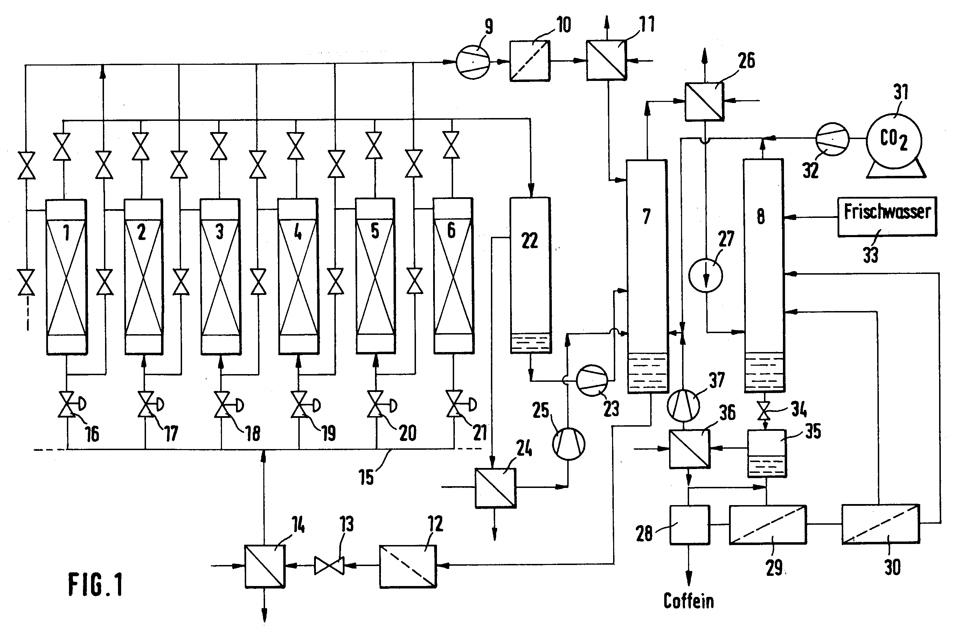

Die vorliegende Erfindung unterscheidet sich in vorteilhafter Weise von der DE-OS 3713953 A1 dadurch, daß statt nassem überkritischen CO₂ oder einer Mischung aus überkritischem CO₂ und wenig Wasser ein mit CO₂ gesättigtes oder übersättigtes flüssiges Lösungsmittel, vorzugsweise Wasser oder eine wässrige Lösung, verwendet wird. Die mit den Rohkaffee-Inhaltsstoffen beladene wässrige Lösung wird in einer nachgeschalteten Vorrichtung, z.B. in der Kolonne 7 in Fig. 1, durch behandeln mit überkritischem CO₂ entcoffeiniert und mit dem CO₂ gesättigt. Durch Entspannung dieser gesättigten Wasser-CO₂-Lösung auf einen niedrigeren Druck entsteht eine übersättigte Wasser-CO₂-Lösung, die in die mit Rohkaffeebohnen befüllten Druckbehälter eingeleitet wird. Damit grenzt sich die vorliegende Erfindung gegen die bekannte Erfindung DE-OS 3713953 A1 dadurch ab, daß zum Aufbau der Druckatmosphäre kein zusätzlicher CO₂-Kreislauf notwendig ist, sondern es werden lediglich die in der Coffeingewinnung auftretenden geringfügigen CO₂-Verluste ersetzt. Weiterhin ist die Rekompression in der vorliegenden Erfindung erheblich wirtschaftlicher, weil die Kompressionsarbeit an einem flüssigen Medium ( Wasser mit CO₂ gesättigt ), welches weitestgehend inkompressibel ist, erfolgt. Weiterhin kann die Entspannung in den Rohkaffee-Extraktionsbehältern erheblich schneller erfolgen als bei DE-OS 3713953 A1, weil hier durch das flüssige Medium im Gegensatz zum gasförmigen Medium bei DE-OS 3713953 A1 ein Einfrieren der Behälterinhalte auch bei schlagartiger Entspannung ausgeschlossen ist.

Das Verfahren kann nach der Erfindung besonders vorteilhaft durchgeführt werden, wenn die Rohkaffeebohnen mit einer mit CO₂ übersättigten und mit den Rohkaffee-Inhaltsstoffen außer Coffein gesättigten wässrigen Lösung bei einer Temperatur von 65 °C bis 90 °C und einem Druck von 30 bar bis 120 bar extrahiert und nach der Entspannung auf 1 bar bis 5 bar mit der weitestgehend coffeinfreien wässrigen Lösung nachgespült werden, die coffeinhaltige wässrige Lösung in einer nachgeschalteten Kolonne mit überkritischem CO₂ bei 65 °C bis 90 °C und 160 bar bis 300 bar entcoffeiniert wird, die mit Coffein beladene überkritische CO₂-Phase in einer weiteren Kolonne durch Auswaschen des Coffeins mit Wasser bei 65 °C bis 90 °C und 160 bar bis 300 bar regeneriert wird, und wenn aus der dabei anfallenden, coffeinhaltigen wässrigen Lösung das Coffein durch z.B. Umkehrosmose gewonnen wird.

Erfindungsgemäß ist eine Sättigung der flüssigen Lösungsmittelphase mit CO₂ besonders geeignet. Es ist aber auch im Sinne dieser Erfindung, daß ein beliebiges Gas oder Gasgemisch verwendet wird, welches sich hinsichtlich seiner thermodynamischen Eigenschaften so verhält, daß es in merklichen Konzentrationen in den mit der Wasser-Coffein-Lösung gefüllten Rohbohnenzellen diffundiert. Auf eine Anfeuchtung der zu entcoffeinierenden Rohkaffeebohnen kann verzichtet werden, wenn Wasser oder eine wässrige Lösung als flüssiges Lösungsmittel verwendet wird. Anderenfalls werden die Kaffeebohnen auf 20 Gew.-% bis 40 Gew.-% angefeuchtet, und dem flüssigen Lösungsmittel wird soviel Wasser zugegeben, daß es nicht zum Austrocknen der Rohkaffeebohnen kommen kann.
Die Rohkaffeebohnen werden mit dem mit CO₂ gesättigten flüssigen Lösungsmittel bei dem erhöhtem Druck je nach Kaffeesorte und gewünschtem Entcoffeinierungsgrad eine bis sechs Stunden extrahiert. Die Entspannung der Druckatmosphäre kann schlagartig erfolgen, da ein Einfrieren der Kaffeebohnen ausgeschloßen ist. Obwohl bei diesem Entspannungsvorgang gasförmiges Kohlendioxid frei wird, tritt überraschenderweise kein Aufschäumen der Flüssigphase auf.

Der Gegenstand der Erfindung wird anhand von Ausführungsbeispielen näher erläutert.

In Fig. 1 ist schematisch der Ablauf einer Variante des Verfahrens zur Herstellung von entcoffeiniertem Rohkaffee dargestellt. Die Rohkaffeebohnen werden je nach Kaffeesorte mit Wasserdampf auf 20 Gew.-% bis 40 Gew.-% angefeuchtet und dann in die Druckbehälter 1 bis 6 eingegeben. Die hier dargestellten Druckbehälter sind in Kaskade geschaltet, so daß ein quasikontinuierlicher Betrieb und damit eine quasikontinuierliche Entcoffeinierung des Rohkaffees möglich wird. Hierdurch wird auch eine vereinfachte Verfahrensführung beim Anfeuchten der Kaffeebohnen und beim anschliessenden Zurücktrocknen dadurch erreicht, daß diese Prozessschritte weitestgehend kontinuierlich erfolgen können und somit lange Lagerzeiten der angefeuchteten Kaffeebohnen vermieden werden. Auch werden dadurch geringere Investitionen für das Anfeuchten, Zwischenlagern und Trocknen im Gegensatz zu einer rein diskontinuierlichen Verfahrensweise aufgewendet. Die in Reihe geschalteten, mit Rohkaffee gefüllten Druckbehälter z.B. 2 bis 5 in Fig. 1 werden so mit dem übersättigten von Coffein befreiten Lösungsmittel beaufschlagt, daß die am weitesten entcoffeinierten Kaffeebohnen mit dem Lösungsmittel zuerst beaufschlagt werden. Das Lösungsmittel strömt sodann in Richtung steigenden Coffeingehalt der Rohkaffeebohnen durch die einzelnen Behälter und verläßt den letzten Behälter mit einer maximal möglichen Coffeinkonzentration.
Die beladene Lösungsmittelphase wird sodann mittels der Pumpe 9 über eine Filterstufe 10 oder eine Zentrifuge ( falls notwendig ) und über einen Wärmetauscher 11 ( falls notwendig ) in die Kolonne 7 eingegeben, wo eine selektive Extraktion des Coffeins bei 200 bar bis 300 bar und 65 °C bis 110 °C sowie eine erneute Sättigung der flüssigen Phase mit CO₂ erfolgt.

Das entcoffeinierte und mit CO₂ gesättigte Lösungsmittel wird am Fusse der Kolonne 7 abgezogen, über die Filterstufe 12 zur Abtrennung gegebenenfalls noch vorhandener Feststoffpartikel geleitet und im nachfolgenden Entspannungsventil 13 auf 30 bis 100 bar entspannt, wodurch eine Mischung aus mit CO₂ gesättigtem Lösungsmittel und CO₂ ( übersättigte Lösung ) entsteht, die im nachgeschalteten Wärmetauscher 14 auf die Betriebstemperatur der Druckbehälter gebracht und in diese eingeleitet wird. Ein Teilstrom 15 der mit CO₂ übersättigten Lösungsmittelphase wird hingegen über ein Regelventil 16 bis 21 in den bereits auf 1 bar bis 5 bar entspannten Druckbehälter z.B. 6 fußseitig eingegeben, wodurch die mit Coffein angereicherte Lösungsmittelphase dort durch frisches Lösungsmittel ersetzt wird. Die z.B. in Behälter 6 ausgetriebene coffeinhaltige Lösungsmittelphase wird über den Entgasungsbehälter 22 und durch die Pumpe 23 in den unteren Teil der Kolonne 7 eingespeist. Das im Entgasungsbehälter 22 freiwerdende CO₂ wird abgesaugt, über den Kondensator 24 verflüssigt und mittels der Flüssiggaspumpe 25 der Kolonne 7 neu zugeführt. Während, wie in Fig. 1 beispielhaft gezeigt wird, die Behälter 2 bis 5 bei einem Druck zwischen 30 und 100 bar entcoffeiniert werden und Behälter 6 bei dem niedrigeren Druck von 1 bis 5 bar gespült wird, wird z.B. der Behälter 1 entleert und mit frischem Rohkaffee beschickt. Nach dem Verschließen von Behälter 1 wird die Flüssigkeit von Behälter 6 nach Abschluß des Spülvorgangs nach Behälter 1 geleitet und gleichzeitig eine Entlüftung von Behälter 1 über ein Entlüftungsventil vorgenommen. Die hierfür notwendigen Leitungen und Ventile sind aus Gründen der besseren Übersicht in Fig. 1 nicht eingezeichnet. Die entkoffeinierten Rohkaffeebohnen in Behälter 6 können gegebenenfalls sodann mit Frischwasser kurz nachgespült werden, um Gewichtsverluste und eventuelle technische Schwierigkeiten bei der nachfolgenden Trocknung zu vermeiden.
Die hier beispielhaft aufgeführte Verfahrensweise ist in beliebiger anderer Reihenfolge oder mit geringer oder größerer Behälterzahl durchführbar, wobei die Quasikontinuität der Verfahrensführung und damit die Wirtschaftlichkeit des Verfahrens mit steigender Behälterzahl steigt, wobei die Gesamtzahl der Behälter eine optimale Größe zwischen 4 und 12 Behälter aufweist. Das kopfseitig aus Kolonne 7 austretende, mit Coffein beladene CO₂ wird über den Wärmetauscher 26 auf die Betriebstemperatur von Kolonne 8 gebracht und mittels der Förderpumpe 27 in den unteren Teil der Kolonne 8 eingegeben und weiter im Kreislauf geführt. In Kolonne 8 wird die mit Coffein beladene CO₂-Phase durch Auswaschen des Coffeins mit Frischwasser 33 und mit aus der Coffeinreingewinnung z.B. durch Umkehrosmose 28 bis 30 anfallendem, weitestgehend coffeinfreiem Restwasser ausgewaschen. Anstelle der Umkehrosmose ist auch eine andere Coffeingewinnung z.B. durch Verdampfung des Wassers möglich. Die im Verfahren auftretenden geringfügigen CO₂-Verluste werden aus dem CO₂-Tank 31 über die Pumpe 32 dem CO₂-Kreislauf wieder zugeführt.
Das erfindungsgemässe Verfahren zeichnet sich letzlich auch dadurch aus, daß der für die Entcoffeinierung des Rohkaffees notwendige Flüssigkreislauf ( zwischen Kolonne 7 und Behältern 1 bis 6 ), der zudem mit den Kaffeeinhaltstoffen gesättigt ist und der für die Coffeingewinnung notwendige Wasserkreislauf ( zwischen Kolonne 8, Umkehrosmose 28 bis 30 sowie Frischwasser 33 ) über eine überkritische CO₂-Phase ( zwischen Kolonne 7 und Kolonne 8 ) gekoppelt ist. Durch die hohe Selektivität des überkritischen Kohlendioxids für Coffein wird gleichzeitig eine nennenswerte Verschleppung und Verlust der für die Aromabildung beim Rösten des Rohkaffees wichtigen Inhaltstoffe verhindert.
Die Kolonnen 7 und 8 werden vozugsweise isobar und isotherm betrieben. Durch eine geeignete Temperaturführung ist aber auch eine Übertragung von Wasser von einer kolonne in die andere möglich. So können z.B. die in den Autoklaven 1 bis 6 gegebenenfalls auftretenden Wasserverluste durch eine höhere Temperaturführung von Kolonne 8 ausgeglichen werden. Wird Kolonne 8 hingegen bei einer niedrigeren Tempereatur betrieben, so kann die Lösungsmittelphase in Kolonne 7 aufkonzentriert werden. Das in der Umkehrosmose bei der Coffeingewinnung anfallende CO₂ wird nach der Entspannung über Ventil 34 aus dem nachfolgenden Entgasungsbehälter 35 abgezogen, im Kondensator 36 verflüssigt und mit Pumpe 37 auf den Druck in Kolonne 7 gebracht und dort eingeleitet.

Beispielsweise wurden nach dem Verfahrensschema nach Fig.1 1000 g ungeröstete Kaffeebohnen mit einer Naturfeuchte von 8 Gew.-% und einem Coffeingehalt von 1,27 Gew.-% i.Tr. ( i.Tr.= bezogen auf Trockensubstanz ) in einem 2,2 l Druckbehälter gegeben. Bei einer Temperatur von 85 °C und einem Druck von 110 bar wurden sie mit einem weitestgehend coffeinfreien, mit CO₂ übersättigten wässrigen Rohkaffee-Extrakt, der 22 Gew.-% gelöste Kaffeefeststoffe enthielt, für vier Stunden bei einem Lösungsmittelstrom von 4 kg/h extrahiert. Anschließend wurde die Druckatmosphäre schlagartig auf 1 bar entspannt, und die Rohkaffeebohnen wurden mit dem Rohkaffee-Extrakt für eine weitere Stunde nachextrahiert, wobei der Massenstrom hier 2 kg/h betrug. Der entcoffeinierte Rohkaffee-Extrakt, der vorher durch wiederholte Auslaugung von frischen Rohkaffeebohnen bei 85 °C zuerst mit frischem und dann mit der mit den Rohkaffee-Inhaltsstoffen beladenen wässrigen Lösung gewonnen und in einer Sprühkolonne durch Behandeln mit überkritischem CO₂ bei 85 °C und 250 bar bis auf einen Coffeinrestgehalt von 80 ppm (mg/kg Lösung) Coffein entcoffeiniert wurde, wurde in einer vorgeschalteten Kolonne bei 85 °C und 250 bar mit CO₂ gesättigt, auf den Druck von 110 bar bzw. 1 bar entspannt, wodurch die übersättigte CO₂-Rohkaffee-Extrakt-Lösung entstand, und in den mit Kaffeebohnen befüllten Druckbehälter eingeleitet. Danach wurden die gequollenen und einen Wassergehalt von 51,6 Gew.-% aufweisenden Bohnen zurückgetrocknet. Sie hatten einen Rest-Coffeingehalt von 0,08 Gew.-% i.Tr., entsprechend einem Entcoffeinierungsgrad von 93,7 % .

## Patentansprüche

1. Verfahren zur Entcoffeinierung von Rohkaffee durch selektive Extraktion des Coffeins mit einem unter Normalbedingungen (1 bar; 20°C) flüssigen Lösungsmittel, bei dem
das Coffein von den Rohkaffeebohnen mit einem ersten Lösungsmittel bei einer Temperatur von 20°C bis 110°C und einem Druck von 30 bar bis 300 bar während einer bis einiger Stunden extrahiert wird,
der Druck schlagartig oder in wenigen Minuten auf 1 bar bis 10 bar vermindert wird, und dabei die Rohkaffeebohnen entspannt werden,
die entspannten Rohkaffeebohnen mit einem zweitem Lösungsmittel für einige Minuten bis zwei Stunden nachgespült werden,
der Vorgang der selektiven Coffeinextraktion mit dem ersten Lösungsmittel bei erhöhtem Druck und der Vorgang des Entspannens und des Nachspülens der Rohkaffeebohnen mit dem zweiten Lösungsmittel bei einem niedrigeren Druck gegebenenfalls wiederholt wird,
die entspannten Rohkaffeebohnen durch Trocknung auf einen für die nachfolgende Röstung notwendigen Wassergehalt gebracht werden, und
die so entcoffeinierten und vorgetrockneten Rohkaffeebohnen geröstet werden,
dadurch **gekennzeichnet**, daß
zum Extrahieren des Coffeins als erstes Lösungsmittel ein mit Kohlendioxid gesättigtes flüssiges Lösungsmittel verwendet wird, dasselbe flüssige Lösungsmittel als zweites Lösungsmittel zum Nachspülen verwendet wird,
das Coffein aus dem mit Coffein beladenen, flüssigen Lösungsmittel selektiv in einem nachgeschalteten, getrennten Kreislauf mittels überkritischem, CO₂ abgetrennt und in kristallinreiner Form gewonnen wird, und
das unter Druck stehende entcoffeinierte Lösungsmittel, unter Ersetzung der in der Coffeingewinnung auftretende CO₂-Verluste, als Lösungsmittel zur Extraktion von Coffein und zur Nachspülung wiederverwendet wird.

2. Verfahren nach Anspruch 1, dadurch gekennzeichnet, daß die zu entcoffeinierenden Rohkaffeebohnen auf einen Wassergehalt von 20 Gew.-% bis 40 Gew.-% angefeuchtet werden.

3. Verfahren nach einem der Ansprüche 1 und 2, dadurch gekennzeichnet, daß ein mit Kohlendioxid übersättigtes flüssiges Lösungsmittel verwendet wird.

4. Verfahren nach einem der Ansprüche 1 bis 3, dadurch gekennzeichnet, daß Wasser als flüssiges Lösungsmittel verwendet wird.

5. Verfahren nach einem der Ansprüche 1 bis 4, dadurch gekennzeichnet, daß eine wässrige Lösung verwendet wird, die mit den Rohkaffee-Inhaltstoffen außer Coffein gesättigt ist.

6. Verfahren nach einem der Ansprüche 1 bis 5, dadurch gekennzeichnet, daß die Rohkaffeebohnen bei einer Temperatur zwischen 65 °C und 90 °C entcoffeiniert werden.

7. Verfahren nach einem der Ansprüche 1 bis 6, dadurch gekennzeichnet, daß die Rohkaffeebohnen mit dem mit Kohlendioxid gesättigten oder übersättigten und weitestgehend coffeinfreien Wasser oder wässrigen Lösungsmittel bei einem Druck von 30 bar bis 120 bar extrahiert und nach dem schnellen Entspannen bei 1 bar bis 5 bar mit dem gleichen Lösungsmittel nachgespült werden.

8. Verfahren nach einem der Ansprüche 1 bis 7, dadurch gekennzeichnet, daß für die Durchführung des Gesamtprozesses mehrere Druckautoklaven in Kaskadenschaltung verwendet werden.

9. Verfahren nach den Ansprüchen 1 bis 8, dadurch gekennzeichnet, daß die mit den Rohkaffee-Inhaltstoffen einschließlich Coffein beladene wässrige Lösung in einer nachgeschalteten Vorrichtung, z.B. Kolonne 7 in Fig. 1, mit überkritischem Kohlendioxid bei einer Temperatur von 65 °C bis 90 °C und einem Druck von 160 bar bis 300 bar selektiv entcoffeiniert und in die mit Rohkaffee befüllten Druckautoklaven erneut geleitet wird.

10. Verfahren nach einem der Ansprüche 1 bis 9, dadurch gekennzeichnet, daß das mit Coffein beladene, z.B. aus dem Kopf der Kolonne 7 ( Fig. 1 ) kommende überkritische Kohlendioxid in einer weiteren Vorrichtung, z.B. Kolonne 8 in Fig. 1, bei einer Temperatur von 65 °C bis 90 °C und einem Druck von 160 bar bis 300 bar durch Auswaschen des Coffeins mit Wasser regeneriert und der ersten Vorrichtung, z.B. dem unteren Teil der Kolonne 7 in Fig. 1, erneut zugeführt wird.

11. Verfahren nach einem der Ansprüche 1 bis 10, dadurch gekennzeichnet, daß die mit Coffein beladene wässrige Lösung aus der Vorrichtung zum Auswaschen des Coffeins aus dem überkritischen Kohlendioxid, z.B. aus dem Sumpf der Kolonne 8 in Fig. 1, abgezogen und in einer Umkehrosmoseanlage in ein Konzentrat mit 4 Gew.-% bis 6 Gew.-% Coffein und ein Permeat mit 30 ppm bis 200 ppm ( mg/kg Lösung ) Coffein aufgetrennt wird, daß aus dem Konzentrat durch Abkühlen auf 0 °C bis 5 °C das auskristallisierende Coffein gewonnen und die Mutterlauge der Umkehrosmoseanlage zugeführt wird, und daß das Permeat zum Auswaschen des Koffeins aus dem überkritischen Kohlendioxid wieder verwendet wird, indem es z.B. der Kolonne 8 ( Fig. 1 ) in der Mitte oder im unteren Bereich zugeführt wird.

12. Verfahren nach einem der Ansprüche 1 bis 10, dadurch gekennzeichnet, daß die Vorrichtungen zur Entcoffeinierung des mit den Rohkaffee-Inhaltstoffen beladenen flüssigen Lösungsmittels, z.B. Kolonne 7 in Fig. 1, und zum Regenerieren des mit Coffein beladenen überkritischen Kohlendioxids, z.B. Kolonne 8 in Fig. 1, bei dem gleichen Druck, aber bei verschiedenen Temperaturen betrieben werden, so daß eine Übertragung von Wasser von einer Vorrichtung in die andere bewirkt wird.

13. Verfahren nach einem der Ansprüche 1 bis 12, dadurch gekennzeichnet, daß die entcoffeinierten und zurückgetrockneten Rohkaffeebohnen geröstet werden.

14. Verfahren nach einem der Ansprüche 1 bis 13, dadurch gekennzeichnet, daß zur Sättigung der flüssigen Lösungsmittelphase neben Kohlendioxid ein beliebiges anderes Gas oder Gasgemisch verwendet wird, welches sich hinsichtlich seiner thermodynamischen Eigenschaften so verhält, daß es in merklichen Konzentrationen in den mit Wasser-Coffein-Lösung gefüllten Rohbohnenzellen diffundiert.

## Claims

1. Process for the decaffeination of raw coffee by selective extraction of caffeine with a solvent which is liquid under normal conditions (1 bar; 20°C), in which
- the caffeine is extracted from the raw coffee beans with a first solvent at a temperature of from 20°C to 110°C and a pressure of from 30 bar to 300 bar for one to a few hours,
- the pressure is reduced suddenly or in a few minutes to 1 bar to 10 bar, and the raw coffee beans are relieved,
- the relieved raw coffee beans are subsequently rinsed with a second solvent for a few minutes up to two hours,
- the process of selective caffeine extraction with the first solvent at increased pressure and the process of relieving and subsequent rinsing of the raw coffee beans with the second solvent at a lower pressure is repeated if necessary,
- the relieved raw coffee beans are brought by drying to a water content necessary for the subsequent roasting, and
- the raw coffee beans thus decaffeinated and pre-dried are roasted,
characterised in that
- a liquid solvent saturated with carbon dioxide is used as the first solvent to extract the caffeine, the same liquid solvent being used as the second solvent for subsequent rinsing,
- the caffeine is selectively separated from the liquid solvent charged with caffeine in a subsequently connected separate cycle by means of supercritical CO₂ and recovered in pure crystalline form, and
- the decaffeinated solvent under pressure is used again as solvent for the extraction of caffeine and for the subsequent rinsing, by replacing the CO₂ losses occurring in the caffeine recovery.

2. Process according to claim 1, characterised in that the raw coffee beans to be decaffeinated are moistened to a water content of from 20% by weight to 40% by weight.

3. Process according to one of claims 1 and 2, characterised in that a liquid solvent supersaturated with carbon dioxide is used.

4. Process according to one of claims 1 to 3, characterised in that water is used as the liquid solvent.

5. Process according to one of claims 1 to 4 characterised in that an aqueous solution is used which is saturated with raw coffee components other than caffeine.

6. Process according to one of claims 1 to 5, characterised in that the raw coffee beans are decaffeinated at a temperature between 65°C and 90°C.

7. Process according to one of claims 1 to 6, characterised in that the raw coffee beans are extracted with the water or aqueous solvent which is as caffeine-free as possible and saturated or supersaturated with carbon dioxide, at a pressure of from 30 bar to 120 bar, and after the fast relief are subsequently rinsed at 1 bar to 5 bar with the same solvent.

8. Process according to one of claims 1 to 7, characterised in that several pressure autoclaves in cascade circuit are used to carry out the entire process.

9. Process according to claims 1 to 8, characterised in that the aqueous solution charged with raw coffee components including caffeine is selectively decaffeinated in a subsequently connected device, e.g. column 7 in Fig. 1, with supercritical carbon dioxide at a temperature of from 65°C to 90°C and a pressure of from 160 bar to 300 bar, and again conveyed into the pressure autoclaves filled with raw coffee.

10. Process according to one of claims 1 to 9, characterised in that the supercritical carbon dioxide charged with caffeine, e.g. coming from the head of the column 7 (Fig. 1) is regenerated in a further device, e.g. column 8 in Fig. 1, at a temperature of from 65°C to 90°C and a pressure of from 160 bar to 300 bar by washing out the caffeine with water, and again conveyed to the first device, e.g. to the lower part of column 7 in Fig. 1.

11. Process according to one of claims 1 to 10, characterised in that the aqueous solution charged with caffeine is drawn off from the device for washing out the caffeine from the supercritical carbon dioxide, e.g. from the sump of column 8 in Fig. 1, and separated in a reverse osmosis installation into a concentrate with 4% by weight to 6% by weight caffeine and a permeate with 30 ppm to 200 ppm (mg/kg solution) caffeine, in that the crystallising caffeine is recovered from the concentrate by cooling to from 0°C to 5°C and the mother liquor is conveyed to the reverse osmosis installation, and in that the permeate is used again for washing the caffeine out of the supercritical carbon dioxide by being conveyed, e.g. to column 8 (Fig. 1) in the middle or in the lower region.

12. Process according to one of claims 1 to 10, characterised in that the devices for decaffeinating the liquid solvent charged with the raw coffee components, e.g. column 7 in Fig. 1, and for regenerating the supercritical carbon dioxide charged with caffeine, e.g. column 8 in Fig. 1, are operated at the same pressure but at different temperatures so that transfer of water from one device to the other is effected.

13. Process according to one of claims 1 to 12, characterised in that the decaffeinated and re-dried raw coffee beans are roasted.

14. Process according to one of claims 1 to 13, characterised in that to saturate the liquid solvent phase any other gas or gas mixture, apart from carbon dioxide, is used which behaves in such a way with respect to its thermodynamic properties that it diffuses in noticeable concentrations in the raw bean cells filled with water-caffeine-solution.

## Revendications

1. Procédé de décaféination de café vert par extraction sélective de la caféine avec un solvant liquide dans les conditions normales <1 bar; 20°C), dans lequel
la caféine est extraite des grains de café vert avec un premier solvant, à une température de 20 à 110°C et sous une pression de 30 à 300 bar, pendant une à quelques heures,
la pression est abaissée brusquement ou en quelques minutes à 1-10 bar et les grains de café vert sont ainsi détendus,
les grains de café vert détendus sont rincés avec un deuxième solvant pendant quelques minutes à 2 h,
le processus d'extraction sélective de la caféine avec le premier solvant sous pression élevée et le processus de détente et de rinçage des grains de café vert avec le deuxième solvant sous pression réduite sont éventuellement répétés,
les grains de café vert détendus sont amenés par séchage à une teneur en eau nécessaire pour l'opération suivante de torréfaction, et
les grains de café vert ainsi décaféinés et préséchés sont torréfiés,
caractérisé en ce que
un solvant liquide saturé d'anhydride carbonique est utilisé comme premier solvant pour l'extraction de la caféine et le même solvant liquide est utilisé comme deuxième solvant pour le rinçage,
la caféine est séparée sélectivement du solvant liquide chargé de caféine, dans un circuit séparé disposé en aval, au moyen de CO₂ surcritique, et elle est récupérée sous forme pure cristalline, et
le solvant décaféiné qui est sous pression est réutilisé, avec remplacement des pertes de CO₂ qui se sont produites dans la récupération de la caféine, comme solvant pour l'extraction de caféine et pour le rinçage.

2. Procédé selon la revendication 1, caractérisé en ce que les grains de café vert à décaféiner sont humidifiés à une teneur en eau de 20 à 40% en poids.

3. Procédé selon la revendication 1 ou 2, caractérisé en ce qu'on utilise un solvant liquide sursaturé d'anhydride carbonique.

4. Procédé selon l'une quelconque des revendications 1 à 3, caractérisé en ce qu'on utilise de l'eau comme solvant liquide.

5. Procédé selon l'une quelconque des revendications 1 à 4, caractérise en ce qu'on utilise une solution aqueuse qui est saturée des constituants du café vert en dehors de la caféine.

6. Procédé selon l'une quelconque des revendications 1 à 5, caractérisé en ce que les grains de café vert sont décaféinés à une température comprise entre 65°C et 90°C.

7. Procédé selon l'une quelconque des revendications 1 à 6, caractérisé en ce que les grains de café vert sont extraits avec l'eau ou le solvant aqueux saturés ou sursatures d'anhydride carbonique et très largement dépourvus de caféine, sous une pression de 30 à 120 bar, et ils sont rincés à la suite de la détente rapide, sous une pression de 1 à 5 bar, avec le même solvant.

8. Procédé selon l'une quelconque des revendications 1 à 7, caractérisé en ce qu'on utilise, pour l'exécution de l'ensemble du procédé, plusieurs autoclaves sous pression disposés en cascade.

9. Procédé selon l'une quelconque des revendications 1 à 8, caractérisé en ce que la solution aqueuse chargée des constituants du café vert, y compris de la caféine, est décaféinée sélectivement, dans un dispositif disposé en aval, par exemple une colonne (7 sur la fig. 1), avec de l'anhydride carbonique surcritique, à une température de 65 à 90°C et sous une pression de 160 à 300 bar, et elle est envoyée de nouveau dans les autoclaves sous pression remplis de café vert.

10. Procédé selon l'une quelconque des revendications 1 à 9, caractérisé en ce que l'anhydride carbonique surcritique chargé de caféine, provenant par exemple de la tête de la colonne (7) (fig. 1), est régénéré dans un autre dispositif, par exemple une colonne (8 sur la fig. 1), à une température de 65 à 90°C et sous une pression de 160 à 300 bar, par extraction de la caféine par lavage à l'eau, et il est renvoyé dans le premier dispositif, par exemple à la partie inférieure de la colonne (7 sur la fig. 1).

11. Procédé selon l'une quelconque des revendications 1 à 10, caractérisé en ce que la solution aqueuse chargée de caféine est évacuée du dispositif où s'effectue l'extraction de la caféine par lavage à partir de l'anhydride carbonique surcritique, par exemple du bas de la colonne (8 sur la fig. 1), et elle est séparée, dans une installation d'osmose inverse, en un concentré contenant 4 à 6% en poids de caféine et un perméat contenant 30 à 200 ppm (mg/kg de solution) de caféine, en ce que la caféine qui se sépare à l'état cristallin est récupérée dans le concentré par refroidissement a 0-5°C et la liqueur mère est envoyée dans l'installation d'osmose inverse, et en ce que le perméat est réutilisé pour l'extraction de la caféine par lavage à partir de l'anhydride carbonique surcritique, en étant envoyé par exemple dans la colonne (8) (fig. 1), au milieu ou dans la région inférieure de celle-ci.

12. Procédé selon l'une quelconque des revendications 1 à 10, caractérisé en ce que les dispositifs pour la décaféination du solvant liquide chargé des constituants du café vert, par exemple la colonne (7 sur la fig. 1), et pour la régénération de l'anhydride carbonique surcritique chargé de caféine, par exemple la colonne (8 sur la fig. 1), sont exploités sous la même pression, mais à des températures différentes, de sorte qu'il se produit un transfert d'eau d'un dispositif à l'autre.

13. Procédé selon l'une quelconque des revendications 1 à 12, caractérisé en ce que les grains de café vert décaféinés et séchés sont torréfiés.

14. Procédé selon l'une quelconque des revendications 1 à 13, caractérisé en ce qu'il est utilisé, en dehors d'anhydride carbonique, pour la saturation de la phase solvante liquide, un quelconque autre gaz ou mélange de gaz qui se comporte, eu égard à ses propriétés thermodynamiques, de sorte qu'il diffuse dans des concentrations notables dans les cellules des grains verts remplies de solution eau/caféine.
